# EUROPÄISCHE PATENTSCHRIFT

(11) **EP 1 757 253 B1**
(45) Veröffentlichungstag und Bekanntmachung des Hinweises auf die Patenterteilung: **19.11.2008**
(21) Anmeldenummer: 05018390.4
(22) Anmeldetag: 24.08.2005
(51) Int. Cl.: A61F 9/013

(54) **Mikrochirurgisches Schneidinstrument für ophthalmologische Behandlungen**
Microsurgical cutting instrument for opthalmic treatments
Instrument de coupe de microchirurgie opthalmique

(43) Veröffentlichungstag der Anmeldung: 28.02.2007
(73) Patentinhaber: WaveLight Laser Technologie AG, 91058 Erlangen (DE)
(72) Erfinder: Donitzky, Christof, 90542 Eckental (DE); Aziret, Sinan, 90441 Nürnberg (DE)
(74) Vertreter: von Hellfeld, Axel

(56) Entgegenhaltungen:
- EP-A- 0 442 156
- US-A1- 2003 060 840
- US-B1- 6 605 099

## Beschreibung

Die Erfindung betrifft ein mikrochirurgisches Schneidinstrument für ophthalmologische Behandlungen, umfassend
- eine auf das Auge aufzusetzende und relativ zu diesem fixierbare Instrumentengrundkörpereinheit,
- einen an der Instrumentengrundkörpereinheit relativ zu dieser in einer Vorschubrichtung beweglich gehaltenen oder halterbaren Schneidkopf,
- eine in einer Aufnahme des Schneidkopfs relativ zu diesem in einer zur Vorschubrichtung quer verlaufenden Lateralrichtung beweglich aufnehmbare Schneidklingeneinheit, und
- Antriebsmittel zum Antrieb des Schneidkopfs in der Vorschubrichtung und zum oszillierenden Antrieb der Schneidklingeneinheit in der Lateralrichtung.

Zur Behebung von Fehlsichtigkeiten niederer Ordnung wie etwa Myopie, Hyperopie, Astigmatismus, myoper Astigmatismus und hyperoper Astigmatismus hat sich unter anderem das Verfahren der sogenannten LASIK (LASer In situ Keratomileusis) etabliert. Dabei wird mittels eines als Mikrokeratom bezeichneten mikrochirurgischen Schneidinstruments ein Scheibchen (Flap) bis auf einen als Scharnier (Hinge) dienenden geringen Rest von der Oberfläche der Hornhaut abgetrennt. Dieses Scheibchen wird zur Seite geklappt, wodurch das darunter liegende Hornhautmaterial für eine refraktiv wirkende Laserbehandlung zugänglich wird. Nach der Behandlung wird das Scheibchen wieder zurückgeklappt. Im Rahmen der Laserbehandlung, die üblicherweise mit einem Excimer-Laser bei einer Wellenlänge von beispielsweise 193 nm erfolgt, wird vom Hornhautstroma gemäß einem vorher bestimmten und ggf. während der Behandlung anzupassenden Ablationsprofil Material ablatiert (entfernt). Die hierdurch bewirkte Neuformung der Hornhaut verändert die Brechungseigenschaften der Hornhaut und damit des optischen Systems des Auges insgesamt. Weil wegen des zurückgeklappten Flaps keine nach außen offene Wunde bleibt, ist die Heilung regelmäßig relativ schnell.

Das Schneidinstrument umfasst einen Schneidkopf, der in einer üblicherweise linearen Vorschubrichtung über die Hornhaut bewegt wird. Der Schneidkopf nimmt eine Schneidklingeneinheit mit einer flachen Schneidklinge auf. Wird der Schneidkopf über die Hornhaut bewegt, schneidet die Schneidklinge in die Hornhaut ein und erzeugt dabei den Flap. Es ist gängig, zusätzlich zum Vorschub des Schneidkopfs die Schneidklingeneinheit in laterale Oszillation zu versetzen, also in Richtung quer zur Vorschubrichtung.

Der Schneidkopf des Schneidinstruments ist beweglich an einer Instrumentengrundkörpereinheit gehalten bzw. halterbar (im Fall eines abnehmbaren Schneidkopfs), die auf das Auge aufzusetzen ist und relativ zu dem Auge fixierbar ist. Zur Fixierung am Auge ist es bekannt, die Instrumentengrundkörpereinheit mit einem Saugring auszuführen, der auf den Limbus aufgesetzt wird und sich mittels Vakuum an diesen ansaugt. Hierzu weist die Instrumentengrundkörpereinheit einen Evakuierungsanschluss auf, der über ein Schlauchleitungssystem an eine externe Vakuumpumpe anschließbar ist und über ein in der Instrumentengrundkörpereinheit gebildetes Evakuierungswegssystem mit einer an der augenzugewandten Unterseite des Saugrings vorgesehenen ringförmigen Evakuierungsnut verbunden ist.

Es ist bekannt, zum lateralen Antrieb der Schneidklingeneinheit diese über eine Exzenterkopplung an eine Antriebswelle anzukoppeln, welche ihrerseits mit einer Abtriebswelle eines Elektromotors in antriebsmäßiger Verbindung steht (siehe z.B. EP 0 442 156 A1). Der Elektromotor wird typischerweise so betrieben, dass sich eine Umdrehungszahl der die Schneidklingeneinheit treibenden Antriebswelle im Bereich zwischen 1.000 und 30.000 U/min, insbesondere zwischen 8.000 und 15.000 U/min einstellt. Die Schneidklingeneinheit weist hierbei eine Nut auf, in die ein an der Antriebswelle angeordneter Exzenterzapfen eingreift.

Für die präzise Erzeugung des Flaps ist eine hohe Schneidwirkung des Schneidinstruments erforderlich. Dem wird üblicherweise durch eine hohe Schärfe der verwendeten Schneidklinge Rechnung getragen. Eine zu hohe Schärfe kann jedoch unerwünscht sein, beispielsweise wenn der Schnitt so gesetzt werden soll, dass nur das Epithel abgelöst wird, ohne dabei die Bowmanmembran und das darunter liegende Hornhautstroma zu verletzen.

Aufgabe der Erfindung ist es daher, eine hohe Schneidwirkung eines Schneidinstruments der eingangs bezeichneten Art auf andere Weise als allein über die Schärfe der Schneidklinge zu ermöglichen.

Zur Lösung dieser Ausgabe ist bei einem solchen Schneidinstrument erfindungsgemäß vorgesehen, dass die Antriebsmittel oder/und ihre Kopplung mit der Schneidklingeneinheit derart ausgestaltet sind, dass die Schneidklingeneinheit auf einem überwiegenden Teil ihres lateralen Oszillationshubs eine wenigstens näherungsweise gleichförmige Geschwindigkeit besitzt. Bei der erfindungsgemäßen Lösung wird im Unterschied zu bekannten Lösungen mit sinusförmigem Geschwindigkeitsverlauf eine näherungsweise Konstanz der Geschwindigkeit der Schneidklingeneinheit über wesentliche Teile des Oszillationshubs erhalten, beispielsweise über wenigstens 60%, wenigstens 70% oder sogar wenigstens 80% des Oszillationshubs. Mit anderen Worten kann bei der erfindungsgemäßen Lösung eine Maximalgeschwindigkeit, die bei sinusförmigem Verlauf der Geschwindigkeit nur an einer einzigen Stelle pro Hub der Schneidklingeneinheit auftritt, nämlich exakt in der Mitte des Hubs, über eine wesentliche Strecke des Hubs, insbesondere den größten Teil des Hubs, aufrechterhalten werden. Dies sorgt für eine verbesserte Schneidwirkung, ohne dass hierzu die Schärfe der Schneidklinge erhöht werden müsste. Es ist sogar vorstellbar, dass angesichts der gesteigerten Schneidwirkung die Schneidklinge so stumpf gemacht werden kann, dass eine Verletzung der Bowmanmembran nicht zu erwarten ist, möglicherweise sogar ausgeschlossen ist.

Die Antriebsmittel können gesonderte Elektromotoren für den Antrieb des Schneidkopfs und der Schneidklingeneinheit umfassen, so dass beide Komponenten unabhängig voneinander gesteuert werden können. Die Schneidklingeneinheit kann dabei über eine an sich bekannte Exzenterkopplung mit einem der Elektromotoren antriebsmäßig gekoppelt sein. Zur Erzielung einer näherungsweise konstanten Geschwindigkeit der Schneidklingeneinheit über wesentliche Teile des Oszillationshubs kann dann eine elektronische Motorsteuereinheit des erfindungsgemäßen Schneidinstruments dazu eingerichtet sein, den mit der Schneidklingeneinheit gekoppelten Elektromotor derart zu steuern, dass eine Abtriebswelle dieses Elektromotors in Endbereichen des Oszillationshubs der Schneidklingeneinheit schneller rotiert als in einem Hubmittelbereich.

Die Erfindung wird nachfolgend anhand der beigefügten Zeichnungen weiter erläutert. Es stellen dar:
Figur 1 ein schematisches Blockschaltbild eines ophthalmologischen Mikrokeratoms gemäß einem Ausführungsbeispiel der Erfindung und
Figur 2 ein beispielhaftes Geschwindigkeitsprofil einer Schneidklingeneinheit des Mikrokeratoms der Figur 1.

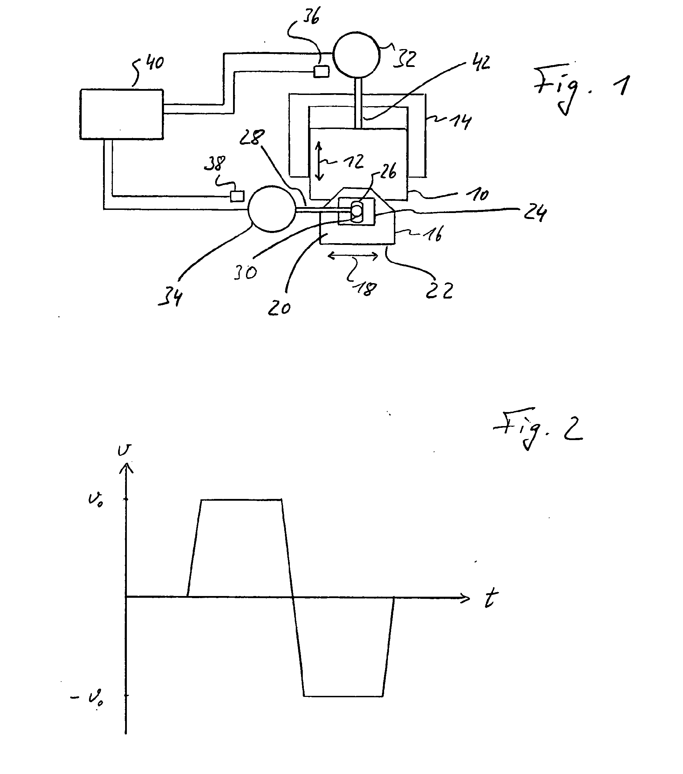

Das in Figur 1 stark schematisiert dargestellte Mikrokeratom umfasst einen Schneidkopf 10, welcher in einer durch einen Doppelpfeil 12 angedeuteten, insbesondere linearen Vorschubrichtung an einer Grundkörpereinheit 14 des Mikrokeratoms geführt ist. Die Grundkörpereinheit 14 weist in nicht näher dargestellter, jedoch an sich bekannter Weise einen Saugring auf, welcher mittels Vakuum am Auge festsaugbar ist. Auf diese Weise wird eine Fixierung der Grundkörpereinheit 14 relativ zum Auge erreicht. Die Grundkörpereinheit 14 ist mit einem Evakuierungsanschluss (nicht dargestellt) versehen, an welchem eine mit einer Vakuumpumpe zu verbindende Schlauchleitung anschließbar ist.

Der Schneidkopf 10 dient als Halter für eine Schneidklingeneinheit 16, welche in einer durch einen weiteren Doppelpfeil 18 angedeuteten Lateralrichtung beweglich an dem Schneidkopf 10 gehalten ist. Hierzu kann der Schneidkopf 10 eine nicht näher dargestellte Aufnahme aufweisen, in die die Schneidklingeneinheit 16 einsteckbar ist und aus der sie nach Gebrauch wieder herausgezogen werden kann. Die Schneidklingeneinheit 16 weist eine beispielsweise aus Edelstahl gefertigte, flache Schneidklinge 20 mit einer Schneidkante 22 an einem vorderen Klingenrand auf. Ein rückwärtiger Klingenrand der Schneidklinge 20 kann zur Führung und Ausrichtung der Schneidklingeneinheit 16 in dem Schneidkopf 10 dienen. Auf einer der Flachseiten der Schneidklinge 20 befindet sich ein Aufsatz 24, welcher fest mit der Schneidklinge 20 verbunden ist. Der Aufsatz 24, der beispielsweise aus Kunststoff gefertigt sein kann, hat einerseits die Funktion, die Handhabung der Schneidklingeneinheit 22 zu erleichtern. Darüber hinaus weist er eine längliche Vertiefung 26 auf, in welche im Betrieb des Mikrokeratoms ein an einer Antriebswellenanordnung 28 angeordneter Exzenterzapfen 30 eingreift.

Zum Antrieb des Schneidkopfs 10 und der Schneidklingeneinheit 16 sind gesonderte Elektromotoren 32, 34 vorgesehen, deren einer, nämlich der Motor 32, dem Schneidkopf 10 zugeordnet ist, während der andere, nämlich der Motor 34, der Schneidklingeneinheit 16 zugeordnet ist. Die Elektromotoren 32, 34 sind Gleichstrommotoren und werden als Servomotoren eingesetzt. Das heißt, jedem der Motoren 32, 34 ist ein Positionsgeber (Encoder) 36 bzw. 38 zugeordnet, der Informationen über die Position bzw. Drehstellung des betreffenden Motors 32, 34 an eine elektronische Motorsteuereinheit 40 liefert. Die Positionsgeber 36, 38 können Absolutwert-Geber oder Inkremental-Geber sein. Abhängig von den von den Gebern 36, 38 gelieferten Positionsinformationen steuert die Motorsteuereinheit 40 den Betrieb der Elektromotoren 32, 34 nach Maßgabe eines gespeicherten Steuerprogramms.

Die Wellenanordnung 28 ist mit dem Elektromotor 34 antriebsmäßig gekoppelt. Eine weitere Wellenanordnung 42 stellt eine antriebsmäßige Verbindung zwischen dem Elektromotor 32 und dem Schneidkopf 10 her. Die beiden Wellenanordnungen 28, 42 können beispielsweise wenigstens auf einem Teil ihrer Länge koaxial ineinander verlaufen. Sie können alternativ auch wenigstens teilweise nebeneinander verlaufen.

Zur Erzeugung eines Hornhautflaps am menschlichen Auge wird die Grundkörpereinheit 14 auf das Auge aufgesetzt und an diesem fixiert. Sodann wird der Schneidkopf 10 unter Steuerung der Motorsteuereinheit 40 mit näherungsweise konstanter Geschwindigkeit in Vorschubrichtung 12 über das Auge bewegt. Gleichzeitig mit dem Vorschub des Schneidkopfs 10 wird die Schneidklingeneinheit 16 unter Steuerung der Motorsteuereinheit 40 in laterale Oszillation (lateral entspricht hier der Erstreckung der Schneidkante 22) versetzt. Nach Erzeugung des Flaps wird der Schneidkopf 10 wieder zurückbewegt, wobei bei dieser Zurückbewegung die Schneidklingeneinheit 16 relativ zum Schneidkopf 10 stillstehen kann. Die Ansteuerung des Elektromotors 34 durch die Motorsteuereinheit 40 ist derart, dass die Geschwindigkeit, mit der die Schneidklingeneinheit 16 ihre Oszillation ausführt, einem näherungsweise trapezförmigen Profil folgt, wie es in Figur 2 beispielhaft dargestellt ist. Gemäß diesem Geschwindigkeitsprofil bewegt sich die Schneidklingeneinheit 16 auf einem Großteil ihres Oszillationshubs mit im wesentlichen konstanter Geschwindigkeit v₀ (in der einen Lateralrichtung) bzw. -v₀ (in entgegengesetzter Lateralrichtung). Erst kurz vor dem Ende des Oszillationshubs, also kurz vor Erreichen des jeweiligen Wendepunkts, fällt die Geschwindigkeit der Schneidklingeneinheit 16 rapide ab und steigt nach Bewegungsumkehr ebenso rapide wieder auf die Maximalgeschwindigkeit v₀ bzw. -v₀ an. Indem so auf dem größten Teil des Oszillationshubs der Schneidklingeneinheit 16 deren Geschwindigkeit im wesentlichen gleichbleibend und maximal ist, ergibt sich eine sehr hohe Schneidwirkung der Schneidklinge 20, die präzise Schnitte ermöglicht, was gleichbedeutend mit der Tatsache ist, dass Flaps mit sauberen Flaprändern und damit reproduzierbarer Größe erzeugt werden können.

Um bei der hier vorliegenden Exzenterkopplung zwischen der Wellenanordnung 28 und der Schneidklingeneinheit 16 das in Figur 2 gezeigte Geschwindigkeitsprofil zu erhalten, steuert die Motorsteuereinheit 40 den Elektromotor 34 solchermaßen, dass die Wellenanordnung 28 nicht mit gleichförmiger Drehgeschwindigkeit rotiert, sondern während jedes Umlaufs zweimal beschleunigt und anschließend wieder abgebremst wird. Die Phasen maximaler Drehgeschwindigkeit der Wellenanordnung 28 entsprechen den Wendepunkten der Schneidklingeneinheit 16 an den Enden des Oszillationshubs. Zur Ermittlung der Position der Schneidklingeneinheit 16 ist es beispielsweise denkbar, dass die Motorsteuereinheit 40 die Stromaufnahme des Elektromotors 34 während einer oder mehreren Umdrehungen bei konstanter Drehgeschwindigkeit ermittelt. Aus dem so gewonnenen Verlauf der Stromaufnahme kann die Steuereinheit 40 feststellen, an welcher Stelle sich die Schneidklingeneinheit 16 entlang ihrer Hubstrecke befindet. Aus den von dem Encoder 38 gelieferten Positionsinformationen kann die Motorsteuereinheit 40 die Drehgeschwindigkeit und Drehbeschleunigung des Elektromotors 34 ermitteln. Sie steuert die Geschwindigkeit bzw. Beschleunigung des Elektromotors 34 dann so, dass sich der gewünschte Geschwindigkeitsverlauf der Schneidklingeneinheit 16 einstellt. Der zur Erzielung des Geschwindigkeitsprofils der Figur 2 benötigte Verlauf der Drehgeschwindigkeit des Elektromotors kann zuvor experimentell ermittelt werden und in der Motorsteuereinheit 40 als Soll abgespeichert sein.

In einer abgewandelten Ausführungsform ist es vorstellbar, die Geschwindigkeit der Schneidklingeneinheit 16 unmittelbar mittels einer am Schneidkopf 10 (oder an der Grundkörpereinheit 14) angeordneten Sensorik zu erfassen und an die Motorsteuereinheit 40 zurückzuführen. Eine solche Sensorik könnte beispielsweise einen oder mehrere optische, induktive oder kapazitive Sensoren enthalten.

Der Antrieb der Schneidklingeneinheit 16 mittels eines Elektromotors ist nur ein Beispiel eines möglichen Antriebskonzepts. Grundsätzlich kann der erfindungsgemäße Geschwindigkeitsverlust der Schneidklingeneinheit auch mit anderen Antriebskonzepten verwirklicht werden, beispielsweise einem Piezoantrieb oder einem Elektromagnetenantrieb.

## Patentansprüche

1. Mikrochirurgisches Schneidinstrument für ophthalmologische Behandlungen, umfassend
- eine auf das Auge aufzusetzende und relativ zu diesem fixierbare Instrumentengrundkörpereinheit (14),
- einen an der Instrumentengrundkörpereinheit relativ zu dieser in einer Vorschubrichtung (12) beweglich gehaltenen oder halterbaren Schneidkopf (10),
- eine in einer Aufnahme des Schneidkopfs relativ zu diesem in einer zur Vorschubrichtung quer verlaufenden Lateralrichtung (18) beweglich aufnehmbare Schneidklingeneinheit (16), und
- Antriebsmittel (32, 34, 40) zum Antrieb des Schneidkopfs in der Vorschubrichtung und zum oszillierenden Antrieb der Schneidklingeneinheit in der Lateralrichtung,
**dadurch gekennzeichnet, dass** die Antriebsmittel oder/und ihre Kopplung mit der Schneidklingeneinheit derart ausgestaltet sind, dass die Schneidklingeneinheit auf einem überwiegenden Teil ihres lateralen Oszillationshubs eine wenigstens näherungsweise gleichförmige Geschwindigkeit besitzt.

2. Schneidinstrument nach Anspruch 1,
**dadurch gekennzeichnet, dass** die Antriebsmittel (32, 34, 40) gesonderte Elektromotoren (32, 34) für den Antrieb des Schneidkopfs (10) und der Schneidklingeneinheit (16) umfassen, dass die Schneidklingeneinheit über eine Exzenterkopplung (30) mit einem (34) der Elektromotoren antriebsmäßig gekoppelt ist und dass eine elektronische Motorsteuereinheit (40) dazu eingerichtet ist, den mit der Schneidklingeneinheit gekoppelten Elektromotor derart zu steuern, dass eine Abtriebswelle dieses Elektromotors in Endbereichen des Oszillationshubs der Schneidklingeneinheit schneller rotiert als in einem Hubmittelbereich.

## Claims

1. A micro-surgical cutting instrument for ophthalmologic treatments, comprising:
- an instrument base body unit (14) to be placed on the eye and fixatable relative thereto,
- a cutting head (10) supported or supportable on the instrument base body unit for movement relative thereto in an advancement direction (12),
- a cutting blade unit (16) receivable in a compartment of the cutting head for movement relative thereto in a lateral direction (18) extending transverse to the advancement direction, and
- drive means (32, 34, 40) for driving the cutting head in the advancement direction and for oscillatingly driving the cutting blade unit in the lateral direction,
**characterized in that** the drive means or/and its coupling to the cutting blade unit is adapted such that the cutting blade unit exhibits at least approximately uniform speed across a major part of its lateral oscillation range.

2. The cutting instrument of claim 1, **characterized in that** the drive means (32, 34, 40) includes separate electric motors (32, 34) for driving the cutting head (10) and the cutting blade unit (16), that the cutting blade unit is drivingly coupled to one (34) of the electric motors via an excentric coupling (30), and that an electronic motor control unit (40) is configured to control the one of the electric motors coupled to the cutting blade unit such that a drive shaft of this electric motor rotates faster in end portions of the oscillating range of the cutting blade unit than in a middle portion of this range.

## Revendications

1. Instrument coupant de microchirurgie pour traitements ophtalmiques, comportant
une unité formant un corps de base (14) de l'instrument, destinée à être mise en place sur l'oeil et pouvant être fixée par rapport à ce dernier,
une tête de coupe (10) maintenue ou pouvant être maintenue sur l'unité formant un corps de base de l'instrument, de manière à pouvoir être déplacée par rapport à cette dernière dans une direction d'avance (12),
une unité à lame coupante (16) pouvant être placée dans un logement de la tête de coupe et déplacée par rapport à cette dernière dans une direction latérale (18) s'étendant transversalement à la direction d'avance, et
des moyens d'entraînement (32, 34, 40) servant à entraîner la tête de coupe dans la direction d'avance et permettant l'entraînement oscillant de l'unité à lame coupante dans la direction latérale,
**caractérisé en ce que** les moyens d'entraînement ou/et leur accouplement à l'unité à lame coupante sont réalisés de telle sorte que l'unité à lame coupante possède une vitesse au moins approximativement uniforme sur une partie prépondérante de sa course d'oscillation latérale.

2. Instrument coupant selon la revendication 1,
**caractérisé en ce que** les moyens d'entraînement (32, 34, 40) comprennent des moteurs électriques individuels (32, 34) pour l'entraînement de la tête de coupe (10) et de l'unité à lame coupante (16), que l'unité à lame coupante est couplée en entraînement à l'un (34) des moteurs électriques par l'intermédiaire d'un accouplement à excentrique (30) et que l'unité de commande électronique (40) du moteur est conçue pour commander le moteur électrique couplé à l'unité à lame coupante de telle sorte qu'un arbre d'entraînement de ce moteur électrique tourne dans des parties terminales de la course d'oscillation de l'unité à lame coupante plus vite que dans une partie médiane de ladite course.
